# DEMANDE DE BREVET EUROPEEN

(11) **EP 1 023 890 A1**
(43) Date de publication de la demande: **02.08.2000**
(21) Numéro de dépôt: 99403268.8
(22) Date de dépôt: 23.12.1999
(51) Int. Cl.: A61K 7/06, A61K 7/48

(54) **Composition anhydre, utilisation en cosmétique, pharmacie ou hygiéne**

(30) Priorité: 26.01.1999 FR 9900829
(71) Demandeur: L'OREAL, 75008 Paris (FR)
(72) Inventeur: Grimm, Sabine, 92290 Châtenay Malabry (FR); Clavel, Euriel, 75013 Paris (FR)
(74) Mandataire: Brédeville, Odile Marie

(57) **Abrégé**

La présente invention a pour objet une composition anhydre, notamment cosmétique, dermatologique, hygiénique ou pharmaceutique, comprenant i°) au moins une phase huileuse comprenant de l'isononanoate d'isononyle et ii°) au moins une phase particulaire comprenant des composés pulvérulents, caractérisée par le fait que l'isononanoate d'isononyle est présent à une teneur d'au moins 10% en poids, par rapport au poids total de la composition, et les composés pulvérulents sont présents à une teneur d'au moins 10 % en poids, par rapport au poids total de la composition.

L'invention se rapporte également aux applications cosmétiques d'une telle composition.

## Description

La présente invention a pour objet des compositions cosmétiques anhydres notamment cosmétique, dermatologique, hygiénique ou pharmaceutique.

Ces compositions peuvent constituer des produits de soin de la peau, y compris le cuir chevelu, et/ou des produits de maquillage de la peau, des muqueuses (lèvres ou intérieurs des paupières), des semi-muqueuses (lèvres), des fibres kératiniques (cheveux, cils, ongles) ou encore des produits de maquillage du corps.

Les compositions de maquillage contenant une phase grasse sont couramment utilisées en cosmétique en raison de leur bonne adhérence à l'épiderme, leur sensation de confort, leur capacité protectrice et leur capacité à former un film imperméable à l'eau. Les produits anhydres de maquillage se présentent en général sous forme solide compacte ou bien sous forme de crème.

Les compositions ayant une consistance onctueuse peuvent être recherchées dans la mesure où elles peuvent être appliquées de façon satisfaisante à l'aide des doigts à partir d'un conditionnement sous forme de pot ou de tube. Elles contiennent généralement des huiles et des cires afin d'obtenir une consistance stable dans le temps.

Toutefois, un des inconvénients de ce type de produit est que, du fait qu'ils comprennent une importante quantité de corps gras, ils laissent sur la peau une sensation de gras, de collant et leur étalement est difficile.

Le but de la présente invention est de fournir une composition anhydre, en particulier sous la forme d'un produit coulé, qui présente des propriétés cosmétiques améliorées, une bonne stabilité. En particulier, il est recherché une composition qui ne donne pas une sensation de gras et de collant à l'application, qui soit néanmoins douce et confortable sur la peau.

Il a maintenant été découvert de façon inattendue et surprenante que par l'emploi d'un ester gras particulier associé à des composés pulvérulents dans des proportions particulières, il était possible d'obtenir des compositions anhydres qui s'étalent facilement sur la peau sans sensation de gras et de collant, qui présentent d'excellentes propriétés cosmétiques, en particulier une très grande douceur ainsi qu'une bonne stabilité.

La présente invention a donc pour objet une composition anhydre, notamment cosmétique, dermatologique, hygiénique ou pharmaceutique, comprenant i°) au moins une phase huileuse comprenant de l'isononanoate d'isononyle et ii°) au moins une phase particulaire comprenant des composés pulvérulents, caractérisée par le fait que l'isononanoate d'isononyle est présent à une teneur d'au moins 10% en poids, par rapport au poids total de la composition, les composés pulvérulents sont présents à une teneur d'au moins 10 % en poids, par rapport au poids total de la composition, et la phase huileuse comprend en outre une huile de silicone, ladite huile de silicone étant présente à une teneur supérieure ou égale à 5% en poids, par rapport au poids total de la composition.

L'invention porte également sur un procédé de traitement non thérapeutique de la peau et/ou du cuir chevelu, notamment un procédé de maquillage, consistant à appliquer sur la peau ou les muqueuses et/ou sur le cuir chevelu une composition telle que définie ci-dessus.

On a constaté que la composition utilisée selon l'invention est particulièrement douce : elle s'applique et s'étale facilement de façon homogène, sans laisser de sensation de gras ou de collant. Elle présente également une texture légère et reste confortable à porter tout au long de la journée.

La composition selon l'invention possède par ailleurs de bonnes qualités sensorielles et cosmétiques, notamment une bonne matité et une bonne couvrance, de l'uniformité et de la tenue.

La composition anhydre selon l'invention permet un maquillage uniforme et homogène.

De plus, appliquée sur la peau, elle présente l'avantage de ne pas migrer dans les plis de la peau et/ou les rides du visage.

Les compositions de l'invention sont des compositions anhydres. Par composition anhydre, on entend une composition comprenant moins de 3 % en poids d'eau, par rapport au poids total de la composition, de préférence moins de 1 % d'eau. Plus préférentiellement encore, la composition ne comprend pas d'eau du tout.

Les compositions selon l'invention comprennent une phase huileuse qui comprend au moins 10% en poids, par rapport au poids total de la composition, d'un ester gras liquide qui est l'isononanoate d'isononyle.

Par phase huileuse, on entend au sens de la présente invention, une phase constituée de corps gras liquides à température ambiante.

De préférence, l'isononanoate d'isononyle est présent dans les compositions selon l'invention à une teneur pouvant aller de 10 à 40% en poids, par rapport au poids total de la composition. De préférence encore, cette teneur va de 15 à 35% en poids, par rapport au poids total de la composition.

Comme produits commerciaux correspondant à l'isononanoate d'isononyle, on peut citer le Wickenol 151 vendu par la société Caschem, le "Dermol 99" vendu par la société Alzo ou encore le "Lanol 99" vendu par la société Seppic.

La composition selon l'invention comprend également une huile de silicone présente à une teneur supérieure ou égale à 5% en poids, par rapport au poids total de la composition. De préférence, la teneur en huile de silicone va de 5 à 55 %, préférentiellement encore de 5 à 25%, en poids, et de préférence encore de 10 à 25% en poids, par rapport au poids total de la composition.

L'huile de silicone utilisable selon l'invention peut être choisie parmi :
- les silicones volatiles cycliques ayant de 3 à 8 atomes de silicium et de préférence 4 à 6, comme par exemple le cyclotétra-diméthylsiloxane, le cyclopentadiméthylsiloxane ou le cyclohexa-diméthylsiloxane; notamment les produits vendus sous les dénominations de "DC Fluid 244", "DC Fluid 245", "DC FLuid 344 et "DC Fluid 345" par la Société Dow Corning,
- les silicones volatiles linéaires ayant de 2 à 9 atomes de silicium, par exemple l'hexaméthyldisiloxane, l'hexylheptaméthyltrisiloxane et l'octylheptaméthyl-trisiloxane;
- les polydiméthylsiloxanes de formule générale suivante : 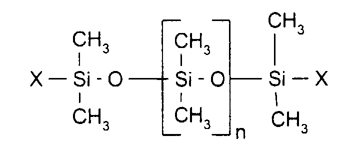
dans laquelle :
- X est -CH₃ ou OH, et
- n est un entier allant de 0 à 2000,
et de préférence ceux dont la viscosité à 25°C est inférieure ou égale à 0,06 m²/s, parmi lesquels on peut citer ceux vendus sous la dénomination "DOW CORNING FLUID 200" par la Société DOW CORNING; on peut également citer notamment les produits vendus sous la dénomination de "AK" par la Société WACKER, "SF" par la Société GENERAL ELECTRIC et "ABIL" par la Société GOLDSCHMIDT, tel que le produit "Abil 10",
- les alkylméthylpolysiloxanes et en particulier les polyalkyl(C₁-C₂₀)siloxanes comme les huiles de silicone phénylées ou la cétyldiméthicone (dénomination CTFA);
- les cyclocopolymères du type diméthylsiloxane/méthylalkyl-siloxane, tels que la "silicone FZ 3109", vendue par la Société UNION CARBIDE, qui est un cyclocopolymère diméthylsiloxane/méthyloctyl-siloxane;
- les gommes de silicone qui sont des polysiloxanes éventuellement phénylés ou hydroxylés, de masse moléculaire élevée, de l'ordre de 200 000 à 1 000 000 et de viscosité supérieure à 500 000 mPa.s, lorsqu'elles sont dissoutes dans un solvant tel qu'une huile polydiméthylsiloxane ou polyphénylsiloxane, ou une cyclométhicone,
- les résines de silicone comprenant une combinaison des unités R₃SiO_{1/2} , R₂SiO_{2/2} , RSiO_{3/2} et SiO_{4/2}, dans lesquelles R est l'hydrogène, un radical alkyle en C₁-C₆ ou un radical phényle, lorsqu'elles sont dissoutes dans un solvant tel qu'une huile polydiméthylsiloxane ou polyphénylsiloxane, ou une cyclométhicone,
- et/ou leurs mélanges.

De préférence, la composition selon l'invention est exempte de phényltriméthicone.

La phase huileuse peut également comprendre des huiles autres que l'isononanoate d'isononyle et les huiles de silicone.

Parmi les autres huiles utilisables selon la présente invention, on peut citer :
- les esters gras autres que l'isononanoate d'isononyle tels que :
   - les esters ramifiés en C₈-C₁₆ comme le néopentanoate d'iso-hexyle,
   - les esters de synthèse comme les huiles de formule R¹COOR² dans laquelle R¹ représente le reste d'un acide gras supérieur comportant de 6 à 29 atomes de carbone et R² représente une chaîne hydrocarbonée contenant de 3 à 30 atomes de carbone, telles que l'huile de Purcellin, le myristate d'isopropyle, le palmitate d'éthyl-2 hexyle, le néopentanoate d'isostéaryle, le myristate d'isopropyle, le stéarate d'isopropyle, le lanolate d'isopropyle, l'isononanoate d'isotridécyle, le stéarate d'octyl-2 dodécyle, l'érucate d'octyl-2-dodécyle, l'isostéarate d'isostéaryle, le propionate d'arachidyle, le benzoate d'octyl-2 dodécyle ; les esters hydroxylés comme l'isostéaryl lactate, l'octylhydroxystéarate, l'hydroxystéarate d'octyldodécyle, le diisostéarylmalate, le citrate de triisocétyle ; les esters de polyols comme le dioctanoate de propylène glycol, le diheptanoate de néopentylglycol, le diisononanoate de diéthylène glycol et les esters du pentaérythritol ;
- les alcools gras ayant de 12 à 16 atomes de carbone comme l'octyldodécanol, le 2-butyloctanol, le 2-hexyldécanol, le 2-undécylpentadécanol, l'alcool oléique ,
- les huiles hydrocarbonées végétales telles que les triglycérides liquides d'acides gras, les triglycérides des acides caprylique/caprique comme ceux vendus par la société Stearineries Dubois ou ceux vendus sous les dénominations Miglyol 810, 812 et 818 par la société Dynamit Nobel ou encore les huiles de germe de blé, de maïs, de tournesol, de karité, de ricin, d'amandes douces, de macadamia, d'abricot, de soja, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat; ces huiles végétales ont la particularité d'être liquides à température égale ou inférieure à 25°C;
- les huiles d'origine minérale, végétale ou animale telles que l'huile de vaseline, l'huile de soja, l'huile de tournesol, l'huile de sésame, l'huile de colza, l'huile d'amande douce, l'huile de macadamia, l'huile de pépins de cassis, le beurre de karité et sa fraction liquide ou le perhydrosqualène ;
- les huiles hydrocarbonées telles que les isoparaffines hydrogénées comme le parléam et notamment les huiles isoparaffiniques volatiles en C₈-C₁₆ comme l'isododécane, l'isodécane et l'isohexadécane,
- les huiles fluorées parmi lesquelles on peut citer les perfluoropolyéthers comme les produits vendus sous la dénomination commerciale "FOMBLIN" par la société MONTEFLUOS, ainsi que les silicones fluorées telles que les trifluorométhyl (C₁-C₄) alkyl diméthicones, par exemple celle vendue sous la dénomination commerciale "X 22819" par la société SHIN ETSU ; et/ou
- leurs mélanges.

La phase huileuse peut par exemple comprendre de 5 à 20% en poids, par rapport au poids total de la composition, d'alcools gras.

De préférence encore, la phase huileuse comprend des esters gras autres que l'isononanoate d'isononyle, ou leurs mélanges, à une teneur pouvant aller de 0,1 à 30 %, préférentiellement encore de 5 à 20%, en poids, par rapport au poids total de la composition.

Dans une forme préférée de réalisation de l'invention, la phase huileuse est constituée uniquement d'isononanoate d'isononyle, d'esters gras et d'huile de silicone et/ou leurs mélanges.

De préférence encore, l'isononanoate d'isononyle est présent à une teneur d'au moins 15% en poids, de préférence d'au moins 30% en poids, par rapport au poids total de la phase huileuse.

Les compositions selon l'invention comprennent également une phase particulaire comprenant des composés pulvérulents choisis parmi les pigments et/ou les nacres et/ou les charges et/ou leurs mélanges habituellement utilisés dans les compositions cosmétiques.

Les composés pulvérulents sont présents à une teneur d'au moins 10% en poids, par rapport au poids total de la composition.

Les charges, qui peuvent être présentes dans la composition à raison de 5-40 % en poids, par rapport au poids total de la composition, peuvent être minérales ou de synthèse, lamellaires ou non lamellaires. On peut citer le talc, le mica, la silice, le kaolin, le Téflon (polytétrafluoroéthylène), l'amidon, la nacre naturelle, le nitrure de bore, les microsphères telles que l'Expancel (Nobel Industrie), les microéponges telles que le polytrap (Dow Corning). On peut également utiliser des charges sphériques telles que les poudres de polyéthylène, les poudres de Nylon, les microbilles de résine de silicone (Tospearls de Toshiba), les microsphères de silice.

De préférence, on utilise les microsphères, notamment de silice, du téflon, du nylon, du talc, du mica et/ou du kaolin.

Dans une forme préférée de réalisation de l'invention, les charges sont présentes à une teneur de 10 à 25% en poids, par rapport au poids total de la composition.

Les pigments peuvent être présents dans la composition à une teneur allant de 0,1 à 30 % en poids, par rapport au poids total de la composition. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré et/ou leurs mélanges. Parmi les pigments organiques, on peut citer le noir de carbone, et les laques de baryum, strontium, calcium, aluminium et/ou leurs mélanges. Les pigments peuvent également présenter une surface hydrophobe ou peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères, notamment des polyéthylènes et/ou des acides aminés.

Parmi les pigments enrobés, on peut citer notamment les pigments vendus sous la dénomination de « SA » par la société Miyoshi (pigments enrobés PDMS).

Les pigments ainsi enrobés peuvent être incorporés dans la composition selon l'invention en une proportion comprise entre 0,1 et 30 % en poids par rapport au poids total de la composition.

Dans une forme préférée de réalisation de l'invention, les pigments sont présents à une teneur d'au moins 2% en poids, de préférence 8 à 15% par rapport au poids total de la composition.

Les nacres peuvent être présentes dans la composition à raison de 0-30% en poids, de préférence 10 à 20% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les compositions selon l'invention peuvent également comprendre des corps gras autres que les huiles citées ci-dessus, comme des cires ou encore des corps gras pâteux. Par cires on entend un corps gras solide à température ambiante.

On peut définir les corps gras pâteux à l'aide d'au moins une des propriétés physico-chimiques suivantes :
- une viscosité de 0,1 à 40 Pa.s (1 à 400 poises), mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

A titre de cires pouvant être utilisées selon l'invention, on peut citer :
- les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, de polytétrafluoroéthylène et les cires obtenues par synthèse de Fisher-Tropsch,
- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la C₃₀-C₄₅ alkyl méthicone vendue sous la dénomination commerciale "AMS C 30" par DOW CORNING,
- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀ vendu sous la dénomination commerciale "KESTER WAX K82H" par la société KOSTER KEUNEN,
- et/ou leurs mélanges.

De préférence, on utilisera les cires de polyéthylène, les cires microcristallines, les cires de carnauba, l'huile de jojoba hydrogénée, les cires de candellila et/ou leurs mélanges.

De préférence, les cires sont présentes à une teneur d'au moins 5% en poids, de préférence encore de 6 à 15%, par rapport au poids total de la composition.

Ces corps gras peuvent en particulier être choisis de manière variée par l'homme du métier afin de préparer une composition ayant les propriétés souhaitées, par exemple en consistance ou en texture.

Parmi les autres adjuvants liposolubles que l'on peut incorporer à la composition, on peut citer les filtres U.V. lipophiles, les vitamines et autres actifs cosmétiques lipophiles, les antioxydants et les parfums, les céramides.

La composition selon l'invention peut comprendre en outre un milieu cosmétiquement, pharmaceutiquement ou hygiéniquement acceptable. Elle peut comprendre alors tout additif usuellement utilisé dans le domaine cosmétique, pharmaceutique ou hygiénique, tels que des antioxydants, des colorants, des parfums, des huiles essentielles, des conservateurs, des actifs cosmétiques, des hydratants, des vitamines, des sphingolipides, des polymères liposolubles notamment hydrocarbonés, tels que le polybutène, les polyalkylènes, les polyacrylates et les polymères siliconés compatibles avec les corps gras.

Ces additifs peuvent être présents dans la composition à raison de 0-15% en poids.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous la forme d'un produit cosmétique et notamment sous la forme d'un produit de soin pour le corps et/ou le visage et/ou le cuir chevelu ou bien encore d'un produit de maquillage, en particulier un fond de teint, un anticerne, un fard à joues ou à paupières, un eye-liner, un mascara ou un rouge à lèvres, un produit de maquillage pour le corps.

Les compositions selon l'invention peuvent se présenter sous la forme d'une crème fluide, d'un stick ou sous la forme d'une pâte souple.

Les compositions selon l'invention sont réalisées selon les méthodes classiques de préparation des compositions anhydres bien connues de l'homme du métier. De préférence, les compositions selon l'invention sont des produits coulés fabriqués par fusion des cires puis ajout des autres composants de la composition et enfin coulage dans une coupelle.

De préférence, la composition selon l'invention est un fond de teint coulé.

L'invention sera mieux illustrée à l'aide des exemples suivants. Dans tous ces exemples, les quantités sont données en pourcentages de poids, par rapport au poids total de la composition.

### EXEMPLE 1 : comparatif

La Demanderesse a réalisé les compositions suivantes :
- isononanoate d'isononyle x %
- palmitate d'éthyl-2 hexyle qsp 100%
- lanolate d'isopropyle 5%
- cires 6 %
- pigments 22 %
- poudre de nylon 16%
- cire de PTFE 7%
- huile de silicone (polydiméthylsiloxane) 8%
avec :

| **Composition** | **% d'isononanoate d'isononyle** |
|---|---|
| Composition A (invention) | 10 |
| Composition B (comparatif) | 5 |

L'ajustement à 100% se faisant sur le pourcentage de palmitate d'éthyl-2 hexyle.

Les compositions A et B peuvent par exemple être des fonds de teint. Elles ont été préparées selon le mode préparatoire suivant : les pigments sont broyés dans la silicone. Les cires sont ensuite chauffées jusqu'à fusion. On ajoute aux cires, tout en continuant à chauffer, les esters gras, les pigments broyés dans la silicone puis les charges. On coule enfin le tout dans une coupelle.

La composition A est plus facile à étaler; elle est plus douce plus facile à maquiller que la composition B. En outre sa texture est plus fine et elle est plus agréable.

### EXEMPLE 2 : comparatif

La Demanderesse a réalisé les compositions C selon l'invention et D, E et F non conformes à l'invention (identiques à C mais dans lesquelles l'isononanoate d'isononyle a été remplacé par un autre ester huileux) suivantes :

### Composition :

- ester huileux 30 %
- palmitate d'éthyl-2 hexyle 7%
- lanolate d'isopropyle 5%
- cires végétales 6 %
- pigments 22 %
- poudre de nylon 16%
- cire de PTFE 7%
- huile de silicone (polydiméthylsiloxane) 7%
avec :

| Composition | Ester huileux |
|---|---|
| Composition C (invention) | isononanoate d'isononyle |
| Composition D (comparatif) | isononanoate d'isotridécyle |
| Composition E (comparatif) | palmitate de 2-éthyl hexyle |
| Composition F (comparatif) | néopentanoate d'isostéaryle |

Les compositions C à F ont été réalisées selon le même mode préparatoire que dans l'exemple 1.

La composition C est très douce. Elle s'étale particulièrement bien. Le maquillage obtenu avec la composition C est plus naturel, il marque moins les zones sèches et les pores que celui obtenu avec les compositions D à F. L'application de la composition C est moins huileuse, moins collante qu'avec les compositions D à F. La texture de la composition C est plus fine, le produit reste moins en surface qu'avec les compositions D à F.

**EXEMPLE 3 :**

On réalise la composition A de l'exemple 1 en remplaçant 5% de lanolate d'isopropyle par 5% d'huile de silicone (polydiméthylsiloxane). La composition obtenue est douce et s'étale particulièrement bien. Elle ne présente pas de sensation de collant ni de gras à l'application.

## Revendications

1. Composition anhydre, notamment cosmétique, dermatologique, hygiénique ou pharmaceutique, comprenant une phase huileuse comprenant de l'isononanoate d'isononyle et une phase particulaire comprenant des composés pulvérulents, caractérisée par le fait que l'isononanoate d'isononyle est présent à une teneur d'au moins 10% en poids, par rapport au poids total de la composition, les composés pulvérulents sont présents à une teneur d'au moins 10 % en poids, par rapport au poids total de la composition, et la phase huileuse comprend en outre une huile de silicone, ladite huile de silicone étant présente à une teneur supérieure ou égale à 5% en poids, par rapport au poids total de la composition.

2. Composition selon la revendication 1, caractérisée par le fait que l'isononanoate d'isononyle est présent dans la composition à une teneur allant de 10 à 40% en poids, de préférence de 15 à 35% en poids, par rapport au poids total de la composition.

3. Composition selon la revendication 1 ou 2, caractérisée par le fait que la phase huileuse comprend en outre des esters gras autres que l'isononanoate d'isononyle, à une teneur pouvant aller de 0,1 à 30 %, préférentiellement encore de 5 à 20%, en poids, par rapport au poids total de la composition.

4. Composition selon la revendication précédente, caractérisée par le fait que les esters gras sont choisis parmi les esters ramifiés en C₈-C₁₆ comme le néopentanoate d'iso-hexyle, les esters de synthèse comme les huiles de formule R¹COOR² dans laquelle R¹ représente le reste d'un acide gras supérieur comportant de 6 à 29 atomes de carbone et R² représente une chaîne hydrocarbonée contenant de 3 à 30 atomes de carbone, telles que l'huile de Purcellin, le myristate d'isopropyle, le palmitate d'éthyl-2 hexyle, le néopentanoate d'isostéaryle, le myristate d'isopropyle, le stéarate d'isopropyle, le lanolate d'isopropyle, l'isononanoate d'isotridécyle, le stéarate d'octyl-2 dodécyle, l'érucate d'octyl-2-dodécyle, l'isostéarate d'isostéaryle, le propionate d'arachidyle, le benzoate d'octyl-2 dodécyle ; les esters hydroxylés comme l'isostéaryl lactate, l'octylhydroxystéarate, l'hydroxystéarate d'octyldodécyle, le diisostéarylmalate, le citrate de triisocétyle ; les esters de polyols comme le dioctanoate de propylène glycol, le diheptanoate de néopentylglycol, le diisononanoate de diéthylène glycol et les esters du pentaérythritol, et/ou leurs mélanges.

5. Composition selon l'une quelconque des revendications précédentes, caractérisée par le fait que l'huile de silicone est présente à une teneur allant de 5 à 55%, de préférence de 5 à 25%, et de préférence encore de 10 à 25% en poids, par rapport au poids total de la composition.

6. Composition selon l'une quelconque des revendications précédentes, caractérisée par le fait que l'huile de silicone est choisie parmi :
- les silicones volatiles cycliques ayant de 3 à 8 atomes de silicium et de préférence 4 à 6, comme par exemple le cyclotétra-diméthylsiloxane, le cyclopentadiméthylsiloxane ou le cyclohexa-diméthylsiloxane;
- les silicones volatiles linéaires ayant de 2 à 9 atomes de silicium, par exemple l'hexaméthyldisiloxane, l'hexylheptaméthyltrisiloxane et l'octylheptaméthyl-trisiloxane;
- les polydiméthylsiloxanes de formule générale suivante : 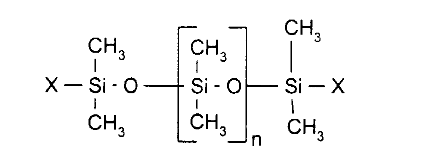
dans laquelle :
- X est -CH₃ ou OH, et
- n est un entier allant de 0 à 2000,
et de préférence ceux dont la viscosité à 25°C est inférieure ou égale à 0,06 m²/s,
- les alkylméthylpolysiloxanes et en particulier les polyalkyl(C₁-C₂₀)siloxanes comme les huiles de silicone phénylées ou la cétyldiméthicone,
- les cyclocopolymères du type diméthylsiloxane/méthylalkyl-siloxane,
- les gommes de silicone qui sont des polysiloxanes éventuellement phénylés ou hydroxylés, de masse moléculaire élevée, de l'ordre de 200 000 à 1 000 000 et de viscosité supérieure à 500 000 mPa.s, lorsqu'elles sont dissoutes dans un solvant tel qu'une huile polydiméthylsiloxane ou polyphénylsiloxane, ou une cyclométhicone,
- les résines de silicone comprenant une combinaison des unités R₃SiO_{1/2} , R₂SiO_{2/2} , RSiO_{3/2} et SiO_{4/2}, dans lesquelles R est l'hydrogène, un radical alkyle en C₁-C₆ ou un radical phényle, lorsqu'elles sont dissoutes dans un solvant tel qu'une huile polydiméthylsiloxane ou polyphénylsiloxane, ou une cyclométhicone,
- et/ou leurs mélanges.

7. Composition selon l'une quelconque des revendications précédentes, caractérisée en ce qu'elle est exempte de phényltriméthicone.

8. Composition selon l'une quelconque des revendications précédentes, caractérisée par le fait que la phase huileuse est constituée uniquement d'isononanoate d'isononyle, d'esters gras et d'huile de silicone et/ou leurs mélanges.

9. Composition selon l'une quelconque des revendications précédentes, caractérisée par le fait que l'isononanoate d'isononyle est présent à une teneur d'au moins 15% en poids, de préférence d'au moins 30% en poids, par rapport au poids total de la phase huileuse.

10. Composition selon l'une quelconque des revendications précédentes, caractérisée par le fait que les composés pulvérulents sont choisis parmi les charges et/ou les pigments et/ou les nacres, et/ou leurs mélanges.

11. Composition selon la revendication 10, caractérisée par le fait que les charges sont choisies parmi les microsphères, notamment de silice, du téflon, du nylon, du talc, du mica et/ou du kaolin.

12. Composition selon la revendication 10 ou 11, caractérisée par le fait que les charges sont présentes à une teneur allant de 5 à 40%, de préférence 10 à 25%, en poids, par rapport au poids total de la composition.

13. Composition selon la revendication 10, caractérisée par le fait que les pigments sont choisis parmi les dioxydes de titane, de zirconium ou de cérium, les oxydes de zinc, de fer ou de chrome, le bleu ferrique, le noir de carbone, les laques de baryum, strontium, calcium, aluminium, les pigments enrobés par des composés siliconés tels que des PDMS et/ou par des polymères, notamment des polyéthylènes et/ou des acides aminés, et :ou leurs mélanges.

14. Composition selon la revendication 13, caractérisée par le fait que les pigments sont présents à une teneur d'au moins 2%, de préférence de 8 à 15%, en poids, par rapport au poids total de la composition.

15. Composition selon l'une quelconque des revendications précédentes, caractérisée par le fait qu'elle comprend en outre des cires.

16. Composition selon la revendication précédente, caractérisée par le fait que les cires sont choisies parmi les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, de polytétrafluoroéthylène et les cires obtenues par synthèse de Fisher-Tropsch,
- les cires de silicone, en particulier les polysiloxanes linéaires substitués; les cires de silicone polyéther, les alkyl ou alkoxy-diméthicones ayant de 16 à 45 atomes de carbone, les alkyl méthicones,
- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀,
- et/ou leurs mélanges.

17. Composition selon la revendication précédente, caractérisée par le fait que les cires sont choisies parmi les cires de polyéthylène, les cires microcristallines, les cires de carnauba, l'huile de jojoba hydrogénée, les cires de candellila et/ou leurs mélanges.

18. Composition selon la revendication 16 ou 17, caractérisée par le fait que les cires sont présentes à une teneur d'au moins 5% en poids, de préférence encore de 6 à 15%, par rapport au poids total de la composition.

19. Composition selon l'une des revendications précédentes, caractérisée par le fait qu'elle se trouve sous la forme d'un produit de maquillage, en particulier un fond de teint, un anticerne, un fard à joues ou à paupières, un eye-liner, un mascara ou un rouge à lèvres, un produit de maquillage pour le corps.

20. Procédé de traitement non thérapeutique de la peau et/ou du cuir chevelu, notamment un procédé de maquillage, consistant à appliquer sur la peau ou les muqueuses et/ou sur le cuir chevelu une composition telle que définie à l'une quelconque des revendications 1 à 19.
